# EUROPEAN PATENT APPLICATION

(11) **EP 4 632 069 A1**
(43) Date of publication of application: **15.10.2025**
(21) Application number: 23900595.2
(22) Date of filing: 04.12.2023
(51) Int. Cl.: C12N 15/117, A61K 31/713, A61K 39/39, A61P 31/00, A61P 35/00, A61P 37/04, A61P 43/00

(54) **NUCLEIC ACID ADJUVANT**

(30) Priority: 05.12.2022 JP 2022193945
(71) Applicant: Aomori Yamada Gakuen Educational Corporation, Aomori-shi, Aomori 030-0943 (JP)
(72) Inventor: SEYA Tsukasa, Aomori-shi, Aomori 030-0943 (JP); MATSUMOTO Misako, Aomori-shi, Aomori 030-0943 (JP)
(74) Representative: Klöckner, Christoph
(86) International application number: PCT/JP2023/043213
(87) International publication number: WO 2024/122480

(57) **Abstract**

The present invention relates to a nucleic acid adjuvant with excellent stability. More specifically, the present invention relates to a nucleic acid composed of a sense strand set forth in SEQ ID NO: 4 and an antisense strand set forth in SEQ ID NO: 5, an adjuvant composition containing the nucleic acid, and a pharmaceutical composition containing the nucleic acid for treating or preventing cancer or an infectious disease.

## Description

### Technical Field

### Related Application:

The present application claims priority to Japanese Patent Application No. 2022-193945 (filed on December 5, 2022), which is incorporated herein by reference.

### Technical Field:

The present invention relates to a nucleic acid composed of a single-stranded oligodeoxynucleotide and a double-stranded RNA, and an adjuvant composition or a pharmaceutical composition containing the nucleic acid.

### Background Art

Activation of a toll-like receptor (TLR) causes the innate immune response of dendritic cells, and induces activation of cytokine production and cellular immunity. Double-stranded RNAs such as poly(I:C) act as ligands for TLR3 and have been considered promising as vaccine adjuvants due to their potent anti-tumor effects; however, their clinical application has been abandoned due to adverse effects such as inflammation and cytokine storm.

It is known that a diRNA (defective interference RNA) derived from measles virus has an adjuvant function (Patent Literature 1). The present inventors reported on a nucleic acid in which a double-stranded RNA having the sequence of the above diRNA is linked to a GpC dinucleotide-containing oligodeoxynucleotide (GpC ODN) that is delivered to endosomes of dendritic cells. According to their reports, such a nucleic acid reaches TLR3 in the endosome, and has a strong adjuvant activity (Patent Literatures 2 to 4). Such an adjuvant nucleic acid induces an NK/CTL-dependent anticancer activity in cancer-grafted murine models without inducing excessive production of inflammatory cytokines (Non Patent Literature 2). Thus, it is expected, as a non-inflammatory nucleic acid adjuvant with less adverse effects, to be applied to vaccines against cancers and infectious diseases.

In order to provide the nucleic acid adjuvant as a pharmaceutical product, the nucleic acid is required to be produced by chemical synthesis in accordance with the GMP criteria. For achieving a high adjuvant activity, the double-stranded RNA moiety preferably has a length of around 100 bases (Patent Literature 4). However, it is not easy to synthesize a long-chain nucleic acid with more than 100 bases, and generally, a longer nucleic acid requires more time and costs for its synthesis. Meanwhile, a nucleic acid in a solution undergoes partial hydrogen bonding, for example, resulting in formation of an undesired complex. It is known that such a complex may change the quality or properties of a nucleic acid pharmaceutical.

### Citation List

### Patent Literatures

Patent Literature 1: WO2008/065752
Patent Literature 2: WO2012/014945
Patent Literature 3: WO2016/088784
Patent Literature 4: WO2018/021400

### Non Patent Literatures

Non Patent Literature 1: Itoh et al., The Journal of Immunology, 2008, vol. 181, No. 8, pp. 5522-5529
Non Patent Literature 2: Matsumoto et al., Nature Communications, 2015, 6:6280
Non Patent Literature 3: Takeda et al., Cell Rep. 2017, 19(9):1874-1887

### Summary of Invention

### Technical Problem

It is a main object of the present invention to provide a nucleic acid adjuvant with safety, efficacy, and stability suitable for practical use.

### Solution to Problem

The present inventors have conducted studies aiming at practical use of a nucleic acid in which GpC ODN is linked to a double-stranded RNA having a sequence of diRNA derived from measles virus (hereinafter, this nucleic acid is collectively referred to as "ARNAX"), and found that an ARNAX having a specific sequence exhibits superior stability to ARNAXs having a similar length.

The present invention has been completed based on the above finding, and relates to a nucleic acid constructed by linking a single-stranded oligodeoxynucleotide (ODN) to be delivered to endosomes of dendritic cells (e.g., single-stranded oligodeoxynucleotide including a GpC dinucleotide (GpC ODN)) to a double-stranded RNA set forth in SEQ ID NO: 3. Specifically, the present invention relates to the following items [1] to [13]:
[1] A nucleic acid constructed by linking a single-stranded oligodeoxynucleotide set forth in SEQ ID NO: 2 to a double-stranded RNA set forth in SEQ ID NO: 3.
   Particularly, a nucleic acid comprising: a sense strand set forth in SEQ ID NO: 4 and an antisense strand set forth in SEQ ID NO: 5.
[2] The nucleic acid according to [1], wherein at least a part of nucleotides constituting the single-stranded oligodeoxynucleotide of the sense strand is phosphorothioate-modified.
[3] An adjuvant composition containing the nucleic acid according to [1] or [2].
[4] A pharmaceutical composition containing the nucleic acid according to [1] or [2].
[5] The pharmaceutical composition according to [4], wherein the composition is used in combination with radiotherapy.
[6] The pharmaceutical composition according to [4], wherein the composition is used in combination with a therapeutic agent for cancer or an infectious disease, preferably an immune checkpoint inhibitor, and more preferably an anti-PD-1 antibody or an anti-PD-L1 antibody.
[7] The pharmaceutical composition according to any of [4] to [6], further containing an antigen, for example, an antigen selected from cancer antigens, bacterial antigens, protozoan antigens, and viral antigens.
[8] The pharmaceutical composition according to any of [4] to [7], for treating or preventing cancer or an infectious disease.
[9] A method of treating or preventing cancer or an infectious disease, the method including administering the nucleic acid according to [1] or [2] to a subject.
[10] The method according to [9], wherein the method is used in combination with radiotherapy.
[11] The method according to [9], wherein the method is used in combination with other therapeutic agent for cancer or an infectious disease, preferably an immune checkpoint inhibitor, and more preferably an anti-PD-1 antibody or an anti-PD-L1 antibody.
[12] The method according to any of [9] to [11], including administering an antigen, for example, an antigen selected from cancer antigens, bacterial antigens, protozoan antigens, and viral antigens to a subject.
[13] Use of the nucleic acid according to [1] or [2] in the manufacture of a pharmaceutical composition for treating or preventing cancer or an infectious disease.
[14] The use according to [13], wherein the pharmaceutical composition is used in combination with radiotherapy.
[15] The use according to [13], wherein the pharmaceutical composition is used in combination with another therapeutic agent for cancer or an infectious disease, preferably an immune checkpoint inhibitor, and more preferably an anti-PD-1 antibody or an anti-PD-L1 antibody.
[16] The use according to any of [13] to [15], wherein the pharmaceutical composition further contains an antigen, for example, an antigen selected from cancer antigens, bacterial antigens, protozoan antigens, and viral antigens.

In other aspects, the present invention further provides the following items [1] to [9]:
[1] A nucleic acid constructed by linking a single-stranded oligodeoxynucleotide including a GpC dinucleotide (GpC ODN) to a double-stranded RNA set forth in SEQ ID NO: 3, wherein the GpC ODN preferably has a length of 15 to 28 bases, more preferably 20 to 28 bases, and even more preferably 24 to 26 bases, for example, 25 bases.
[2] The nucleic acid according to [1], wherein the GpC ODN is a single-stranded oligodeoxynucleotide (ODN) set forth in SEQ ID NO: 2, or an ODN including a part thereof consisting of 20 or more consecutive bases thereof (e.g., 20 or more consecutive bases, preferably 23 or more consecutive bases, and more preferably 24 or more consecutive bases in SEQ ID NO: 2) and having a length of 20 to 28 bases, preferably 24 to 26 bases.
[3] The nucleic acid according to [1] or [2], wherein the single-stranded ODN and the double-stranded RNA are linked to each other via a linker.
[4] The nucleic acid according to [1] or [2], wherein the single-stranded ODN and the double-stranded RNA are directly linked to each other.
[5] The nucleic acid according to any of [1] to [4], wherein the nucleic acid is in a lyophilized form.
[6] The nucleic acid according to [5], wherein the nucleic acid is stable for 12 months at -20°C.
[7] The nucleic acid according to [5] or [6], wherein the nucleic acid shows no decrease in ability of activating IFN β via TLR3 even after preservation for 12 months at-30°C.
[8] An adjuvant composition containing the nucleic acid according to any of [1] to [7].
[9] A pharmaceutical composition containing the nucleic acid according to any of [1] to [7].

### Effects of Invention

The nucleic acid of the present invention is highly stable, and is easily preserved and handled. The nucleic acid of the present invention specifically activates TLR3 and induces NK/CTL without inducing systemic production of inflammatory cytokines. Therefore, the nucleic acid of the present invention is useful for prevention or treatment of cancers and infectious diseases, either alone or in combination with other pharmaceutical(s).

### Brief Description of Drawings

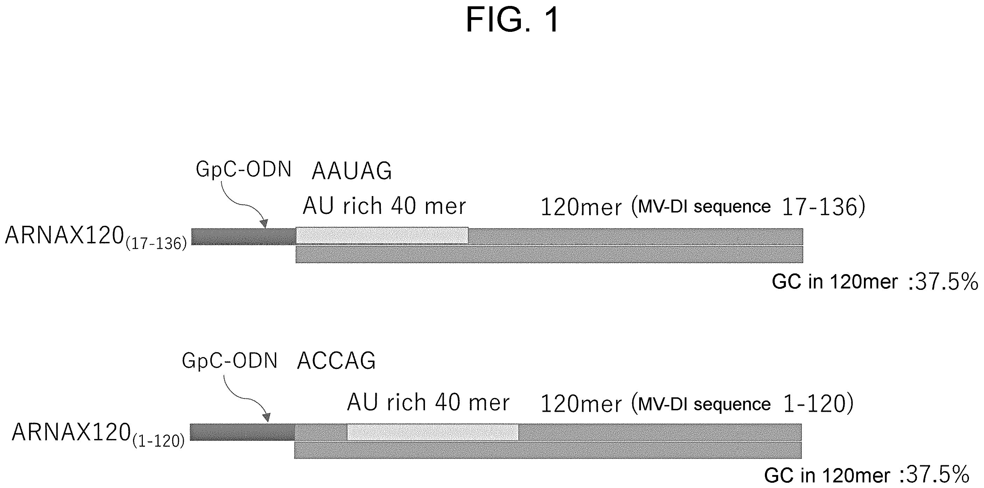

[Figure 1] Figure 1 shows structures of ARNAX120₍₁₇₋₁₃₆₎ and ARNAX120₍₁₋₁₂₀₎.

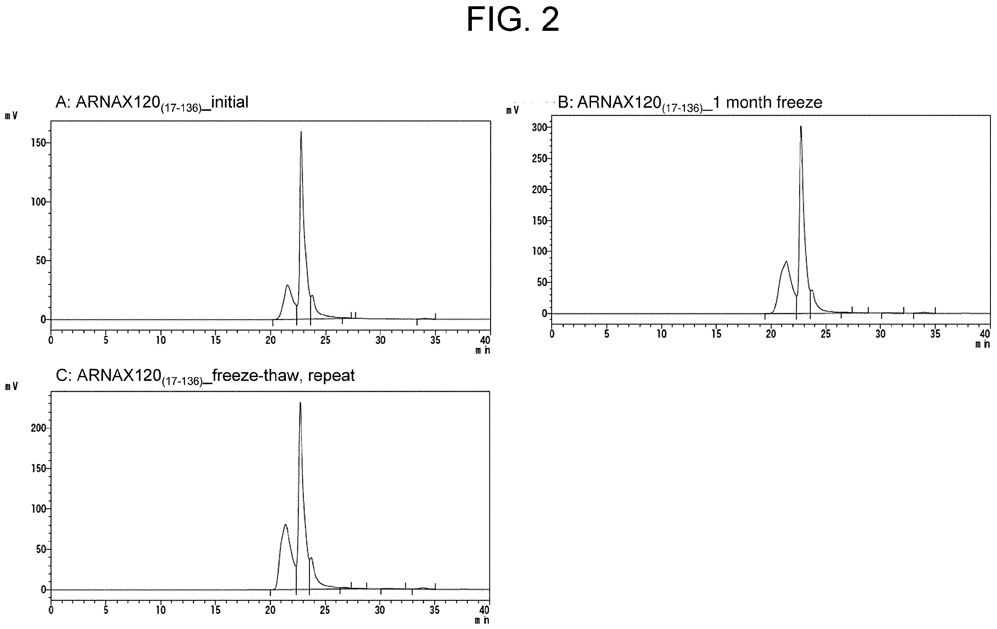

[Figure 2] Figure 2 shows results of SEC-HPLC of a lyophilized product of the ARNAX120₍₁₇₋₁₃₆₎ (A: before freezing (initial), B: after cryopreservation for one month, C: after repeated freeze-thaw cycles).

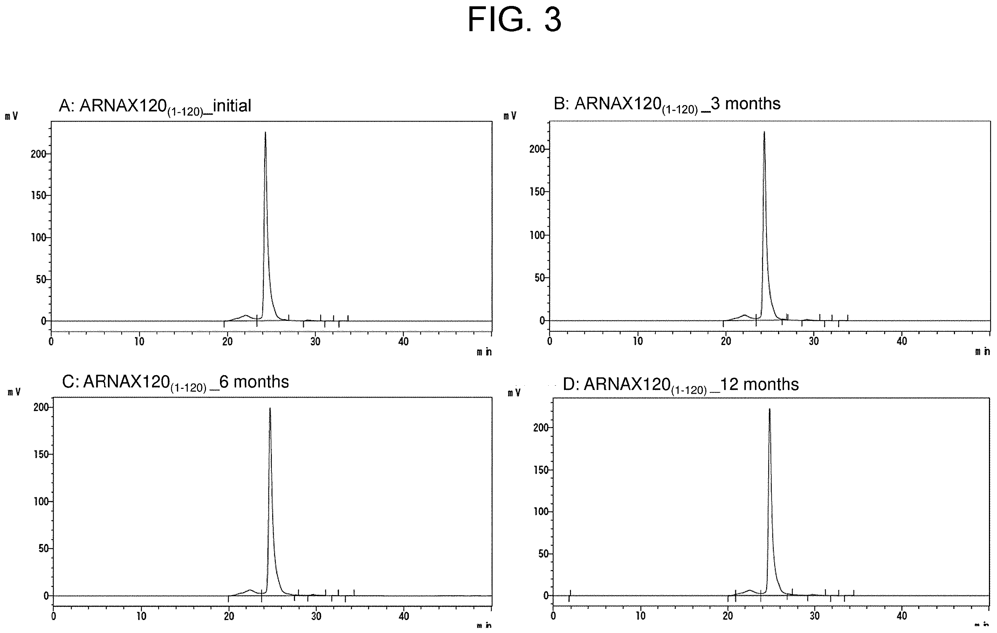

[Figure 3] Figure 3 shows results of SEC-HPLC of a lyophilized product of the ARNAX120₍₁₋₁₂₀₎ (A: before freezing (initial), B: after cryopreservation for three months, C: after cryopreservation for six months, D: after cryopreservation for 12 months).

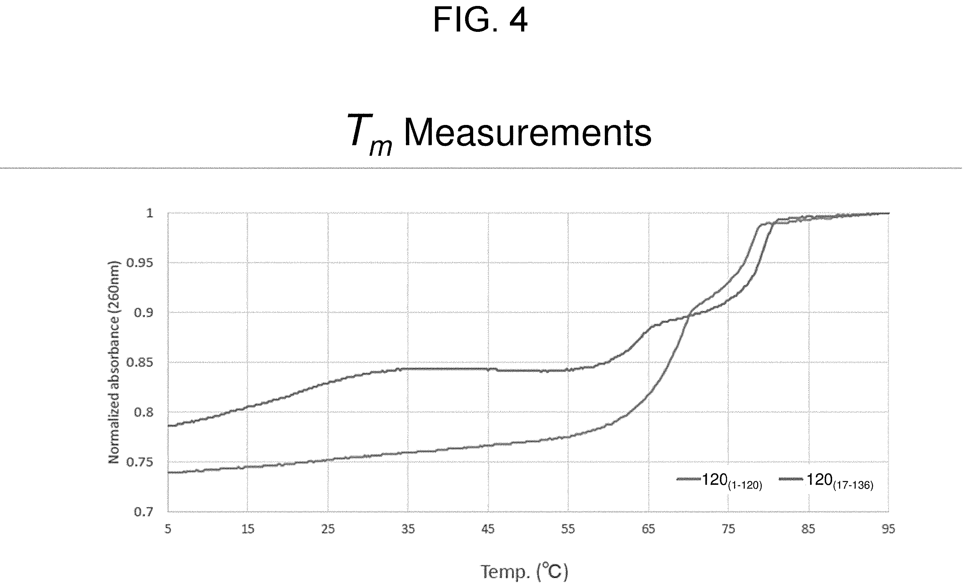

[Figure 4] Figure 4 shows results of measurement of Tm values of the ARNAX120₍₁₇₋₁₃₆₎ and the ARNAX120₍₁₋₁₂₀₎.

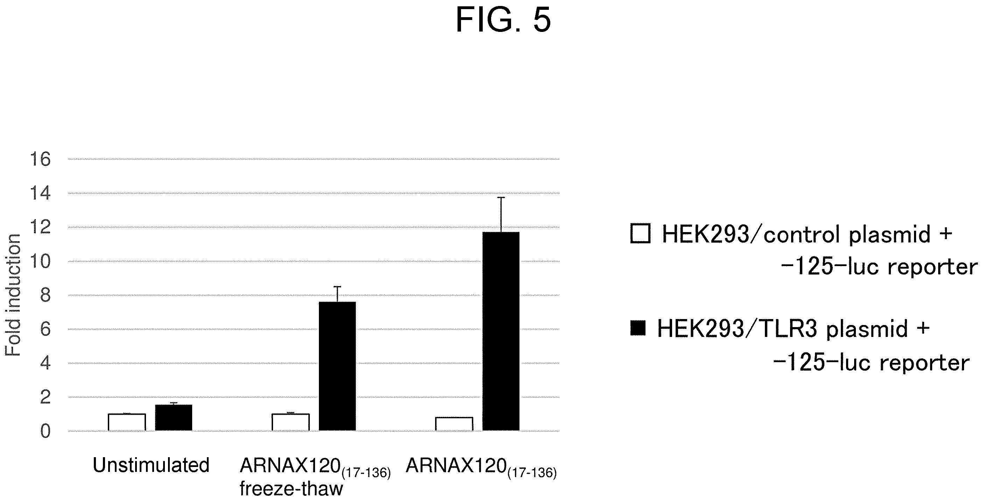

[Figure 5] Figure 5 shows a luciferase activity of the ARNAX120₍₁₇₋₁₃₆₎ (ability of activating IFNβ via TLR3). It shows, from the left, PBS (unstimulated), freeze-thawed ARNAX120₍₁₇₋₁₃₆₎ (10 µg/mL), and non-freeze-thawed ARNAX120₍₁₇₋₁₃₆₎ (10 µg/mL). □ (open bar) indicates control plasmid + -125-luc reporter, and ■ (filled bar) indicates HEK293/TLR3 plasmid + -125-luc reporter.

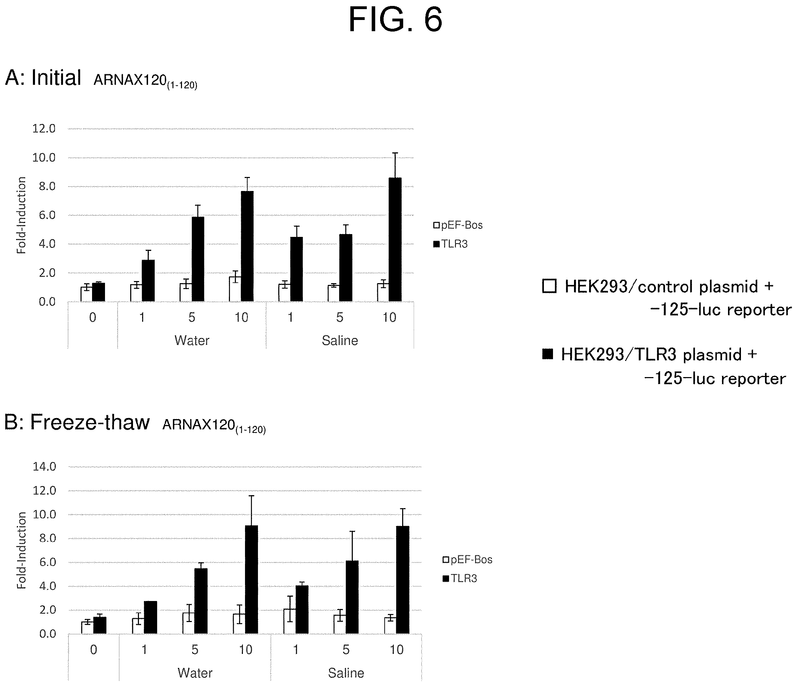

[Figure 6] Figure 6 shows a luciferase activity of the ARNAX120₍₁₋₁₂₀₎ (ability of activating IFNβ via TLR3), where A indicates immediately after dissolution (initial) and B indicates after freeze-thaw (for each, from the left, PBS (unstimulated), water-solved ARNAX120₍₁₋₁₂₀₎ (1, 5, and 10 µg/mL), and saline-solved ARNAX120₍₁₋₁₂₀₎ (1, 5, and 10 µg/mL)). □ (open bar) indicates control plasmid + -125-luc reporter, and ■ (filled bar) indicates HEK293/TLR3 plasmid + -125-luc reporter.

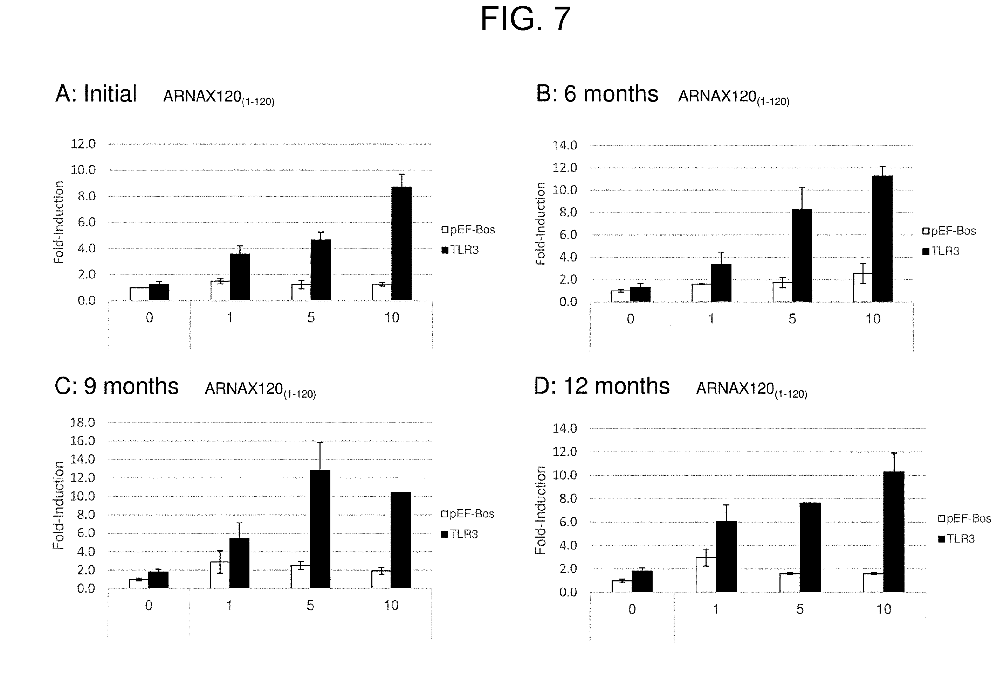

[Figure 7] Figure 7 shows the luciferase activity of the ARNAX120₍₁₋₁₂₀₎ (ability of activating IFNβ via TLR3) after cryopreservation at -30°C (months 0 (initial), 6, 9, and 12). □ (open bar) indicates control plasmid + -125-luc reporter, and ■ (filled bar) indicates HEK293/TLR3 plasmid + -125-luc reporter.

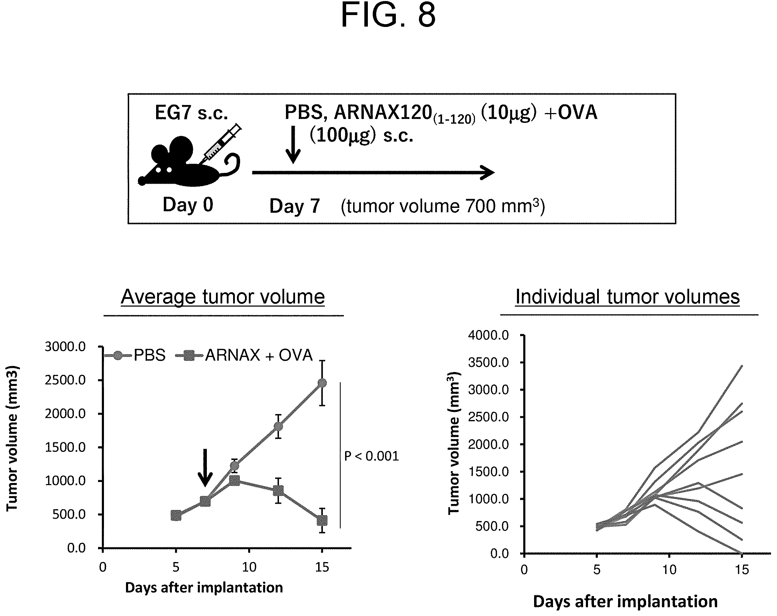

[Figure 8] Figure 8 shows a tumor regression effect by the ARNAX120₍₁₋₁₂₀₎, where the left indicates an average tumor volume and the right indicates individual tumor volumes.

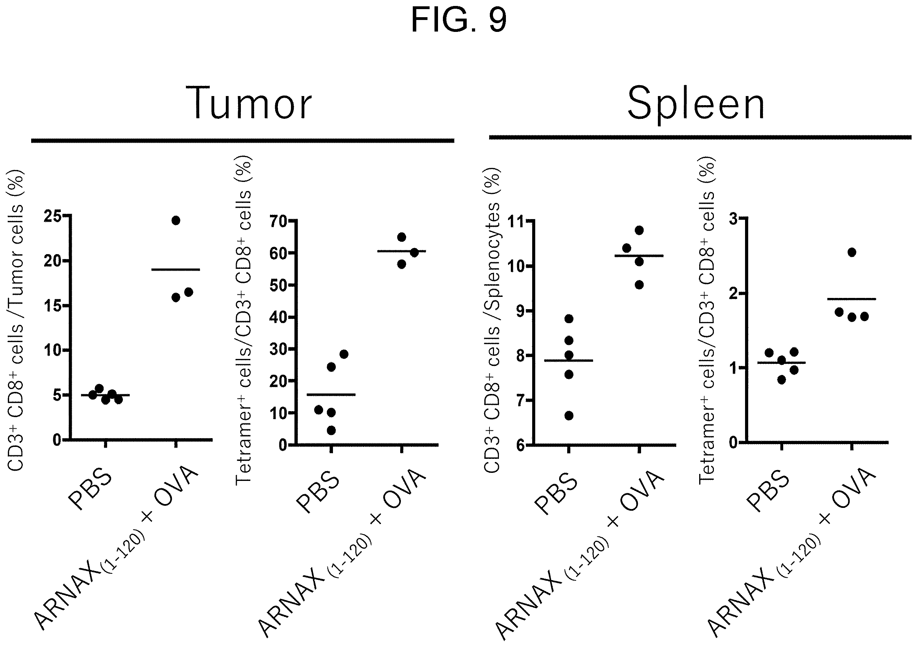

[Figure 9] Figure 9 shows CTL induction by the ARNAX120₍₁₋₁₂₀₎ in the tumor (left) and in the spleen (right). For the tumor, the results for the group with ARNAX₍₁₋₁₂₀₎ + OVA were for three individuals (n=3), because one individual exhibited complete regression.

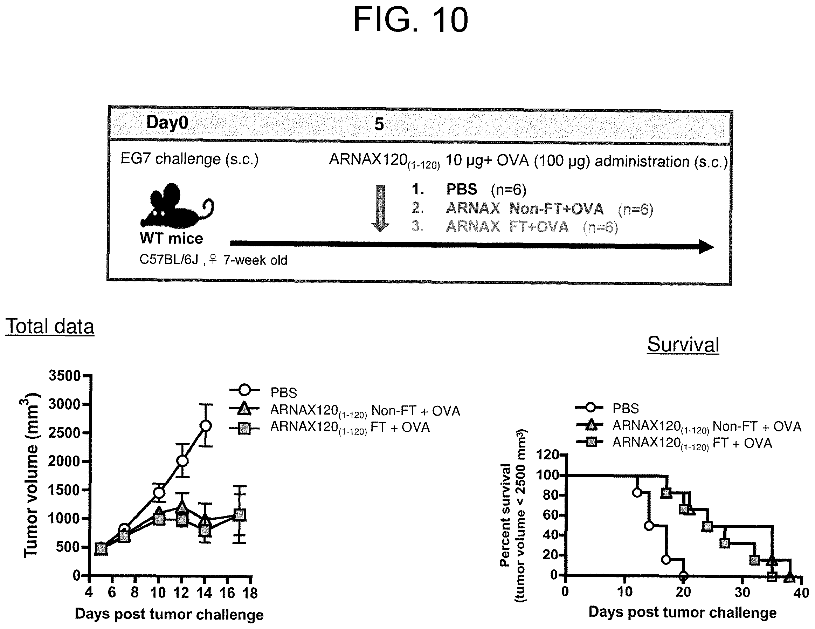

[Figure 10] Figure 10 shows test results for effects of freeze-thawing on the tumor regression effect of the ARNAX120₍₁₋₁₂₀₎, where the left shows the average tumor volume and the right shows the survival rate. For each, ○ (circle) indicates PBS (control), △ (triangle) indicates non-freeze-thawed ARNAX120₍₁₋₁₂₀₎, □ (square) indicates freeze-thawed ARNAX120₍₁₋₁₂₀₎.

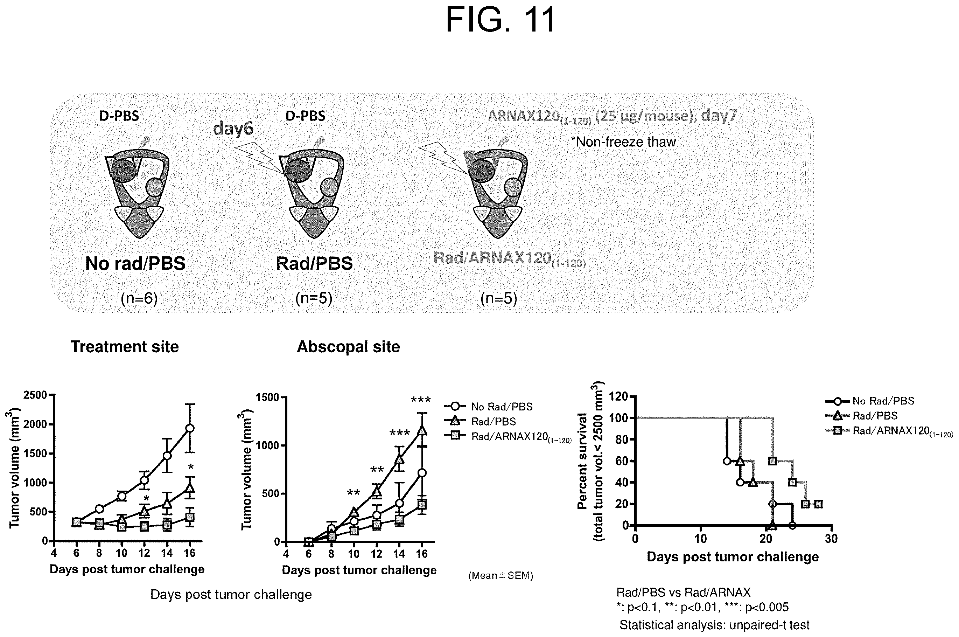

[Figure 11] Figure 11 shows an abscopal effect of the ARNAX120₍₁₋₁₂₀₎, where the left indicates a tumor volume of a treatment area (irradiated area), the center indicates a tumor volume of an abscopal area (unirradiated area), and the right indicates a survival rate. For each, ○ (circle) indicates no irradiation/PBS, △ (triangle) indicates irradiation/PBS, □ (square) indicates irradiation/ARNAX120₍₁₋₁₂₀₎.

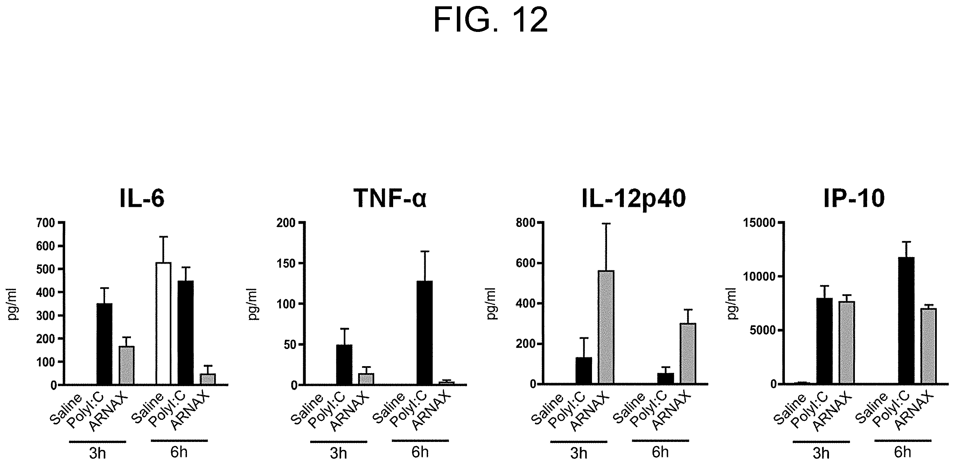

[Figure 12] Figure 12 shows induction of cytokines (IL-6, TNF-α, IL-12p40, and IP-10) caused by subcutaneous administration of the ARNAX120₍₁₋₁₂₀₎. The graphs show serum cytokine levels (pg/ml) at hour 3 and hour 6 (for both time points, the levels are arranged in the order of saline, poly(I:C) (PolyI:C), and ARNAX120₍₁₋₁₂₀₎ (ARNAX) groups, from the left).

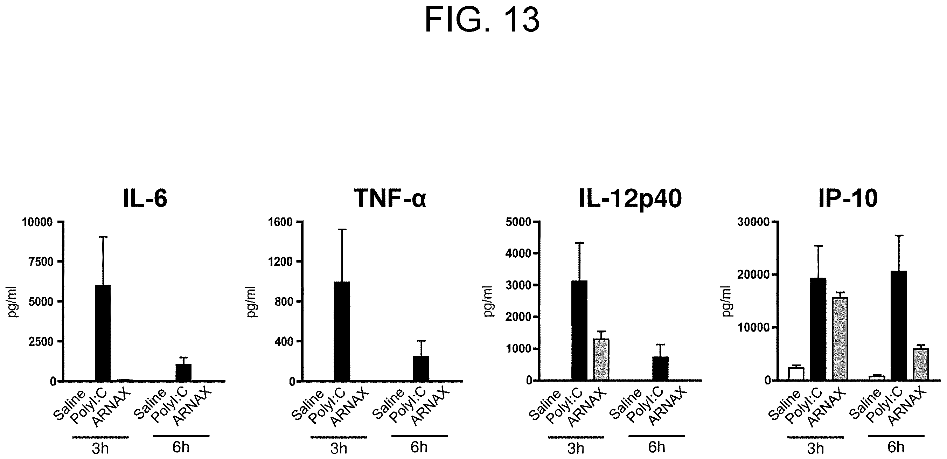

[Figure 13] Figure 13 shows induction of cytokines (IL-6, TNF-α, IL-12p40, and IP-10) caused by intraperitoneal administration of the ARNAX120₍₁₋₁₂₀₎. The graphs show serum cytokine levels (pg/ml) at hour 3 and hour 6 (for both time points, the levels are arranged in the order of saline, poly (I:C) (PolyI:C), and ARNAX120₍₁₋₁₂₀₎ (ARNAX) groups, from the left).

### Description of Embodiments

### 1. Nucleic Acid of the Present Invention (ARNAX)

A nucleic acid of the present invention (ARNAX) having an adjuvant activity is composed of a double-stranded RNA based on a diRNA derived from measles virus; and a single-stranded oligodeoxynucleotide (single-stranded ODN) to be delivered to endosomes. Among the ARNAXs, the present invention relates particularly to the ARNAX120₍₁₋₁₂₀₎ including a double-stranded RNA consisting of 120 bases of positions 1 to 120 in SEQ ID NO: 1.

### (1) Double-stranded RNA

Microorganism-derived double-stranded RNAs are known to activate innate immunity as a TLR3 ligand. Similar phenomenon is also observed in artificially-synthesized double-stranded RNAs (Matsumoto M., and T. Seya. 2008. TLR3: Interferon induction by double-stranded RNA including poly(I:C). Adv. Drug Del. Rev. 60: 805-812.). TLR is a type 1 membrane protein, and recognizes components derived from viruses or bacteria and induces a defense response. TLR3, a member of the Toll-like receptor (TLR) family, recognizes extracellular double-stranded RNA as its ligand and induces diverse cellular responses via TICAM-1.. TLR3 is localized in endosomes in myeloid dendritic cells, while it is localized on cell surfaces and in endosomes in some epithelial cells and macrophages. In both cases, since a TLR3-mediated signal is transduced from the endosomes, the double-stranded RNA is required to be incorporated into the cell.

Microorganism-derived double-stranded RNAs do not act on a human gene. Thus, researches are ongoing in order to use a microorganism-derived double-stranded RNA as a vaccine adjuvant, as it controls the innate immunity as a TLR3 ligand. The "double-stranded RNA" used in the present invention is such an exogenous double-stranded RNA, and activates TLR3 to activate the innate immunity without affecting human endogenous genes.

The Edmonston (ED) strain is an attenuated strain of measles virus. An ED strain-derived diRNA (SEQ ID NO: 1) has been conventionally contained in vaccines (Shingai et al., J Immunol. 2007; 179: 6123-6133), and has established safety in human application. The present inventors found that the diRNA from the ED strain has an adjuvant function (ibid. Itoh et al., The Journal of Immunology, 2008; Matsumoto et al. Nat. Commun. 2015). They also confirmed that a double-stranded RNA prepared based on the above ED-derived diRNA and having a length of 59 to 140 bases was efficiently delivered to endosomes, when linked with a single-stranded ODN described later, to activate only TLR3 without activating intracellular RNA sensors RIG-I or MDA5, and achieve the tumor regression effect (ibid. WO2012/014945 and WO2016/088784). This double-stranded RNA specifically activates TLR3, without a concern of systemic inflammatory cytokine induction, and is therefore excellent as a vaccine adjuvant.

The length of the "double-stranded RNA" is preferably about 50 or more base pairs in order to achieve TLR3 activation potency, preferably a length of about 100 or more bases in order to achieve effective tumor regression, and preferably about 120 or more bases, if possible. However, at present, it is not easy to synthesize a nucleic acid with a length of more than 100 bases. Therefore, it is advantageous for the nucleic acid to have a shorter length, in view of the synthesis thereof.

It is known that a GC content level influences on thermal stability. The present inventors prepared various double-stranded RNAs including a GC-rich region of SEQ ID NO: 1, and confirmed their activity. They also confirmed that nucleic acids including 58 or more consecutive bases among the base sequence of positions 1 to 140 of SEQ ID NO: 1 had an immunoadjuvant activity. However, the GC content is not necessarily correlated with the activity of the ARNAX (since an ED strain-derived diRNA forms a stem loop, the base sequence of positions 1 to 140 forms a complementary strand with the sequence set forth in positions 1017 to 1074).

From the perspective of the synthesis efficiency, it is preferred to use an AU-rich region among the sequence set forth in SEQ ID NO: 1. Examples of such an AU-rich region include positions 17 to 78, positions 101 to 164, and positions 216 to 269 of SEQ ID NO: 1. The present inventors reported that a suitable adjuvant activity and tumor regression effect were exhibited by double-stranded RNAs including 40 bases of positions 17 to 56 of SEQ ID NO: 1 as an AU-rich region, particularly double-stranded RNAs of consecutive 100 to 160 bases in length, preferably 110 to 150 bases in length, and more preferably 120 to 140 bases in length, which include the 40 bases of positions 17 to 56 of SEQ ID NO: 1 (mentioned above).

As described above, in order to put the ARNAX into practical use, various aspects thereof, such as activity, synthesis efficiency, and thermal stability, should be considered. As a nucleic acid adjuvant which is suitable in view of the synthesis efficiency and the activity, the present inventors have so far reported on ARNAX120₍₁₇₋₁₃₆₎ including a double-stranded RNA consisting of 120 bases of positions 17 to 136 of SEQ ID NO: 1 (WO2018/021400). However, this ARNAX120₍₁₇₋₁₃₆₎ was revealed to aggregate during preservation.

The ARNAX120₍₁₋₁₂₀₎ including a double-stranded RNA consisting of 120 bases of positions 1 to 120 of SEQ ID NO: 1 is completely the same as those of the ARNAX120₍₁₇₋₁₃₆₎ in length, GC content (thermal stability), the AU-rich region included (synthesis efficiency), and the sequence of the single-stranded RNA moiety, but it exhibits excellent preservation stability without generating aggregation during cryopreservation, unlike the ARNAX120₍₁₇₋₁₃₆₎. The ARNAX120₍₁₋₁₂₀₎ exhibits an excellent adjuvant activity and tumor regression effect even after the cryopreservation, and thus is suitable for practical use as a pharmaceutical.

### (2) Single-stranded Oligodeoxynucleotide (Single-stranded ODN)

TLR9 recognizes an unmethylated CpG motif of a viral DNA or a bacterial DNA. TLR9-mediated signaling is performed at high efficiency by a short synthetic oligodeoxynucleotide (ODN). This ODN includes a CpG motif characteristic of bacterial and viral genes, and is well-known in the art as "CpG ODN." It is known that the CpG ODN is useful as a strong vaccine adjuvant or antibody production enhancer, because it is delivered to endosomes of dendritic cells and serves as a TLR9 ligand. Meanwhile, the CpG ODN strongly induces MyD88-dependent cytokines (especially IFNα), and causes a Th2 response.

An ODN obtained by replacing CpG in the CpG ODN with GpC, TpC, or CpC does not have the TLR9 agonist activity, despite its endosomal delivery, and thus does not induce an unnecessary immune response. ARNAXs use such an ODN without the TLR9 agonist activity as the single-stranded ODN.

The GpC ODNs without the TLR9 agonist activity are commercially available as a control (as it does not have the TLR9 agonist activity) to the CpG ODN (http://www.invivogen.com/tlr9-agonist). In the synthesis of ARNAX, these ODNs can also be used as the single-stranded ODN.

The single-stranded ODN is preferably 15 or more bases in length, especially 15 to 28 bases in length, for example, 20 to 28 bases in length or 24 to 26 bases in length, from the perspective of the endosomal delivery function. In the present invention, the following GpC ODN with a length of 25 bases is used as the single-stranded ODN:
GpC ODN: tgctgctgcttgcaagcagcttgat (SEQ ID NO: 2).

The single-stranded nucleotide constituting the GpC ODN is preferably modified, from the perspective of the stability (nuclease resistance). Examples of such modification include phosphorothioate modification. As a result of the phosphorothioate modification, the single-stranded ODN enables efficiently endosomal delivery of the nucleic acid without being degraded by nuclease.

### 2. Synthesis of ARNAX

The ARNAX is composed of the "double-stranded RNA" and the "single-stranded ODN" described above. The double-stranded RNA and the single-stranded ODN may be linked with each other either directly or via a suitable linker. The ARNAX120₍₁₋₁₂₀₎ does not include a linker, but a suitable linker may be inserted without imparing the object of the present invention.

The ARNAX can be synthesized as a nucleic acid composed of a single-stranded nucleic acid A (a chimeric nucleic acid of the "single-stranded ODN" and a sense strand of the "double-stranded RNA") and a single-stranded nucleic acid B (an antisense strand of the "double-stranded RNA"). Each of these single-stranded nucleic acids (the sense strand and the antisense strand) can be synthesized in accordance with the method described in WO2016/088784 mentioned above, for example, by synthesizing partial sequences and sequentially ligating them. Of course, the synthesis is not limited to the above method, and may be performed in accordance with a method well-known in the art, by linking the double-stranded RNA to the single-stranded ODN.

Each of the single-stranded nucleic acids constituting the ARNAX preferably has no attached phosphate group at either end. This is because, when administered to a living body in a large amount, a phosphate group remaining at the 5' end activates an intracytoplasmic RIG-I pathway and induces production of large amounts of cytokines to cause adverse effects (Robinson et al. J Natl Cancer Inst. 1976 Sep; 57(3):599-602). When an RNA strand is synthesized by in vitro transcription, a triphosphate is added to the 5' end of the RNA strand. On the other hand, the nucleic acid of the present invention can be produced by chemical synthesis, and thus can be synthesized as a nucleic acid with no phosphate group attached to either the 5' or 3' end.

### 3. Preservation of ARNAX

The synthesized ARNAX is lyophilized, and is cryopreserved until it is used. A cryopreserved product of ARNAX120₍₁₋₁₂₀₎ is stable for at least one year at a temperature of -20°C or lower.

In some embodiments, the ARNAX₍₁₋₁₂₀₎ is preserved in a form dissolved in water (distilled water for injection) or saline, thawed in use, and used in a 1xPBS solution. Also after the freezing and thawing, the ARNAX120₍₁₋₁₂₀₎ is stable, and does not lose its adjuvant activity and antitumor activity. Besides water or saline, the ARNAX120₍₁₋₁₂₀₎ may be preserved in a form dissolved in a buffer solution commonly used in pharmaceutical products, such as a phosphate buffer solution. The pH of the solution (buffer solution) is preferably close to neutral.

The ARNAX₍₁₋₁₂₀₎ in a phosphate buffer solution is stable for two days at a temperature not exceeding 30°C. Thus, it is applicable at room temperature (1 to 30°C) and normal temperature (15 to 25°C).

### 4. Adjuvant Composition

The present invention also provides an adjuvant composition containing the ARNAX. In tumor microenvironments, in addition to tumor cells, myeloid cells that infiltrate the tumor create an immunosuppressive state. Tumor-associated macrophages (TAMs) strongly support the growth, maintenance, and invasion of tumors, and contribute to the formation of a microenvironment favorable for tumors, while myeloid-derived suppressor cells (MDSCs) suppress the activity of antigen-specific T cells. Administration of Poly(I:C) has a TLR3-dependently strong anti-cancer activity, but has a problem of systemic production of IFN-α/β, inflammatory cytokines, via MDA5 expressed in a wide range of cells. In contrast, it is considered that administration of the ARNAX can activate only TLR3, without activating intracellular RLRs such as MD5, and convert cancer suppressor cells in tumors into cancer attacker cells to inhibit the tumor growth. Therefore, the administration of the ARNAX does not induce excessive inflammatory responses, and the ARNAX is useful as a nucleic acid adjuvant with reduced adverse effects.

The ARNAX has the following characteristics: 1) it is efficiently taken up by dendritic cells; 2) it activates only TLR3 and does not activate MDA5/RIG-1; 3) it does not induce systemic production of inflammatory cytokines/type I IFN; 4) it activates dendritic cells and induces NK cells and CTLs; 5) it shows a strong anti-tumor activity; 6) it can inhibit the tumor growth by controlling the microenvironment in the tumor; 7) it can be chemically synthesized in accordance with the GMP criteria; 8) it is expected to have promising applications to vaccines against even viral infectious diseases which cannot be prevented by induction of antibody production (RSV, SARS, etc.). Therefore, the ARNAX is extremely useful as an adjuvant in immunotherapy.

The adjuvant composition of the present invention may also contain a pharmacologically acceptable carrier or additive, in addition to the ARNAX (ARNAX120₍₁₋₁₂₀₎). Examples of such a carrier or additive include, but are not limited to, surfactants, excipients, coloring agents, flavoring agents, preservatives, antioxidants, stabilizers, buffers, suspending agents, isotonizing agents, binders, disintegrating agents, lubricants, flow enhancers, taste masking agents, and the like. Other commonly used carriers can be used as appropriate.

Specifically, aqueous carriers include water, ethanol, polyols (glycerol, propylene glycol, polyethylene glycol, and the like), plant oils such as olive oil, and organic esters such as ethyl oleate.

Examples of non-aqueous carriers can include light anhydrous silicic acid, lactose, crystalline cellulose, mannitol, starch, carmellose calcium, carmellose sodium, hydroxypropyl cellulose, hydroxypropylmethyl cellulose, polyvinyl acetal diethylaminoacetate, polyvinylpyrrolidone, gelatin, medium-chain fatty acid triglyceride, polyoxyethylene hydrogenated castor oil 60, sucrose, carboxymethyl cellulose, corn starch, inorganic salts, and the like.

Examples of the antioxidant include water-soluble antioxidants such as ascorbic acid, cysteine hydrochloride, sodium bisulfate, sodium metabisulfite, and sodium sulfite, fat-soluble antioxidants such as ascorbyl palmitate, butylated hydroxyanisole (BHA), butylated hydroxytoluene (BHT), lecithin, propyl gallate, and α-tocopherol, and metal chelating agents such as citric acid, ethylenediaminetetraacetic acid (EDTA), sorbitol, tartaric acid, and phosphoric acid.

An administration route of the adjuvant composition of the present invention is not particularly limited, but parenteral administration is preferred. Specific examples of the parenteral administration include injection administration, nasal administration, pulmonary administration, transdermal administration (including patches), and the like. Examples of the injection administration can include intravenous injection, intramuscular injection, intraperitoneal injection, and subcutaneous injection. Among them, subcutaneous injection is particularly preferred. The administration method can be appropriately selected depending on the age and symptoms of a patient.

The dose of the adjuvant composition of the present invention is appropriately determined depending on its purpose of use, administration route, and the like. In the case of administration to a human subject, the dose can be selected so that the amount of ARNAX (ARNAX120₍₁₋₁₂₀₎) per administration is in the range of 0.00001 mg to 10 mg per 1 kg body weight, for example. Alternatively, the dose can be selected so that the amount of ARNAX (ARNAX120₍₁₋₁₂₀₎) per patient is in the range of 1 to 100 mg/body, for example. However, the dose of the adjuvant composition of the present invention is not limited to the doses described above.

The adjuvant composition of the present invention may contain an antigen molecule. However, since endogenous antigens are present in the body of a cancer patient or a bacteria-infected patient, the immune effect can be achieved even when the adjuvant nucleic acid is used alone. When the adjuvant nucleic acid alone cannot exhibit a sufficient effect, an antigen molecule may be administered together with the adjuvant, depending on the disease to be treated. The antigen may be in any of the following forms: protein, mRNA, VLP, and whole particle. Examples of the antigen molecule include viral antigens, bacterial antigens, protozoan antigens, cancer antigens, and antigenic components thereof.

Examples of the viral antigen include, for example, viral antigens such as adenovirus, retrovirus, picornavirus, herpesvirus, rotavirus, hantavirus, coronavirus, togavirus, flavivirus, rhabdovirus, paramyxovirus, orthomyxovirus, bunyavirus, arenavirus, reovirus, papillomavirus, parvovirus, poxvirus, hepadnavirus, spongiform virus, HIV, CMV, hepatitis A virus, hepatitis B virus, hepatitis C virus, influenza virus, measles virus, poliovirus, smallpox virus, rubella virus, herpes simplex virus, varicella zoster virus, Epstein-Barr virus, Japanese encephalitis virus, rabies virus, and influenza virus, and combinations thereof.

Examples of the bacterial antigen include bacterial antigens from Bacillus, Escherichia, Listeria, Neisseria, Nocardia, Salmonella, Staphylococcus, Streptococcus, and the like, and combinations thereof.

Examples of the protozoan antigen include protozoan antigens from malaria parasites, Toxoplasma gondii, amoeba protozoa, coccidian protozoa, Isospora protozoa, Giardia protozoa, Cryptosporidium protozoa, cyclospora protozoa, and the like, and combinations thereof.

Examples of the cancer antigen include cancer antigens of leukemia, lymphoma, astrocytoma, glioblastoma, melanoma, breast cancer, lung cancer, head and neck cancer, digestive system tumor, gastric cancer, colon cancer, liver cancer, pancreatic cancer, uterine cancer, ovarian cancer, vaginal cancer, testicular cancer, prostate cancer, penile cancer, bone tumor, vascular tumor, esophageal cancer, rectal cancer, colorectal cancer, pancreatic cancer, galSlbladder cancer, bile duct cancer, laryngeal cancer, bronchial cancer, bladder cancer, kidney cancer, brain tumor, thyroid cancer, Hodgkin's disease, non-Hodgkin's lymphoma, multiple myeloma, and the like, and combinations thereof.

### 5. Pharmaceutical Composition

In addition to the adjuvant effect, the ARNAX may also have a therapeutic effect on cancer, infectious diseases, or the like. The present invention also provides a pharmaceutical composition containing the ARNAX (ARNAX120₍₁₋₁₂₀₎), particularly a pharmaceutical composition for treating cancer or an infectious disease.

Examples of the cancer targeted by the pharmaceutical composition of the present invention include bone cancer, pancreatic cancer, skin cancer, head and neck cancer, melanoma, uterine cancer, ovarian cancer, rectal cancer, anal cancer, gastric cancer, testicular cancer, uterine cancer, fallopian tube carcinoma, endometrial carcinoma, cervical carcinoma, vaginal carcinoma, vulvar carcinoma, Hodgkin's disease, non-Hodgkin's lymphoma, esophageal cancer, small intestine cancer, endocrine system cancer, thyroid cancer, parathyroid cancer, adrenal cancer, soft tissue sarcoma, urethral cancer, penile cancer, acute myeloid leukemia, chronic myeloid leukemia, acute lymphoblastic leukemia, chronic leukemia, acute leukemia, childhood solid cancer, lymphocytic lymphoma, bladder cancer, kidney cancer, ureteral cancer, renal pelvis carcinoma, central nervous system (CNS) tumor, primary CNS lymphoma, tumor angiogenesis, spinal tumor, brain stem glioma, pituitary adenoma, Kaposi's sarcoma, squamous cell carcinoma, planocellular carcinoma, T-cell lymphoma, environmentally-induced tumors, and the like.

Examples of the infectious disease targeted by the pharmaceutical composition of the present invention can include HIV infection (AIDS), SARS (SARS-COV1 and SARS-COV2), hepatitis, herpes, malaria, Leishmaniasis, influenza, dysentery, pneumonia, tuberculosis, sepsis, and the like. In particular, the present invention can be appropriately applied to HIV infection which causes severe immunodeficiency.

The pharmaceutical composition of the present invention may also contain a pharmacologically acceptable carrier or additive, in addition to the ARNAX (ARNAX120₍₁₋₁₂₀₎). Examples of such a carrier or additive include, but are not limited to, surfactants, excipients, coloring agents, flavoring agents, preservatives, antioxidants, stabilizers, buffers, suspending agents, isotonizing agents, binders, disintegrating agents, lubricants, flow enhancers, taste masking agents, and the like. Other commonly used carriers can be used as appropriate. Specific examples of them are as described in the section of the "adjuvant composition."

An administration route of the pharmaceutical composition of the present invention is not particularly limited, but parenteral administration is preferred. Specific examples of the parenteral administration include injection administration, nasal administration, pulmonary administration, transdermal administration, and the like. Examples of the injection administration can include intravenous injection, intramuscular injection, intraperitoneal injection, and subcutaneous injection. The administration method can be appropriately selected depending on the age and symptoms of a patient.

The dose of the pharmaceutical composition of the present invention is appropriately determined depending on its purpose of use, administration route, and the like. In the case of administration to a human subject, the dose can be selected so that the amount of ARNAX (ARNAX120₍₁₋₁₂₀₎) per administration is in the range of 0.00001 mg to 10 mg per 1 kg body weight, for example. Alternatively, the dose can be selected so that the amount of ARNAX (ARNAX120₍₁₋₁₂₀₎) per patient is in the range of 1 to 100 mg/body, for example. However, the dose of the pharmaceutical composition of the present invention is not limited to the doses described above.

The pharmaceutical composition of the present invention may contain an antigen molecule. However, since endogenous antigens are present in the body of a cancer patient or a bacteria-infected patient, the immune effect can be achieved even when the ARNAX is used alone. When the ARNAX alone cannot exhibit a sufficient effect, an antigen molecule may be administered together with the adjuvant, depending on the disease to be treated. Examples of the antigen molecule include viral antigens, bacterial antigens, cancer antigens, and antigenic components thereof. Specific examples of them are as described in the section of the "adjuvant composition."

The pharmaceutical composition of the present invention may also be used in combination with other anticancer agent or anti-infective agent. The present inventors have reported that a synergistic effect can be obtained by combining the ARNAX with an immune checkpoint inhibitor such as an anti-PD-1 antibody or an anti-PD-L1 antibody (ibid., WO2018/021400). While anti-PD-1 antibody or anti-PD-L1 antibody acts at the effector phase of the immune defense mechanism, the ARNAX is believed to act at the priming phase, as it is delivered to the endosomes of dendritic cells, activates TLR3, and induces NK cells and CTLs. Therefore, by combining the ARNAX with an anti-PD-1 antibody or an anti-PD-L1 antibody, the immune defense mechanism can be activated through both the priming phase and the effector phase.

By the action mechanism described above, the above combination of the ARNAX is effective even against cancer for which an anti-PD-1 antibody or anti-PD-L1 antibody alone is ineffective, and more powerfully induces CTL-dependent tumor regression. Furthermore, the above combination of the ARNAX can achieve an effect which cannot be achieved by anti-PD-1 antibody or anti-PD-L1 antibody alone, that is, antibody production and NK cell activation, as well as improvement of the tumor microenvironment. The ARNAX is highly safe and can be safely administered even to elderly patients, and can expand the target of the treatment using an anti-PD-1 antibody or anti-PD-L1 antibody to enhance its effects.

The pharmaceutical composition of the present invention may also be used in combination with radiotherapy. This can improve the effect of the radiotherapy in the treatment of cancer. When the pharmaceutical composition of the present invention is used in combination with the radiotherapy, an abscopal effect is observed in which cancer regression occurs even in unirradiated areas. The abscopal effect refers to an effect of radiotherapy in which cancer regression occurs not only in the irradiated area but also in separated untreated areas. The abscopal effect is believed to be caused as cancer antigen information and the like released from cancer cells destroyed in the irradiated area activates the immune system of separate lesions.

### Examples

Hereinafter, the present invention will be described in more detail with reference to Examples, but the present invention is not limited to the Examples.

### Example 1: Stability of ARNAX120₍₁₇₋₁₃₆₎ and ARNAX120₍₁₋₁₂₀₎ during Cryopreservation

### 1. Formation of Aggregation

Two types of nucleic acids, the ARNAX120₍₁₇₋₁₃₆₎ and the ARNAX120₍₁₋₁₂₀₎ which differ in the sequence of the double-stranded RNA (120-mer) moiety were synthesized (Figure 1). The ARNAX120₍₁₇₋₁₃₆₎ is consisting of the sense strand of SEQ ID NO: 7 and the antisense strand of SEQ ID NO: 8. The ARNAX120₍₁₋₁₂₀₎ is consisting of the sense strand of SEQ ID NO: 4 and the antisense strand of SEQ ID NO: 5. The two nucleic acids have the same sequence of the single-stranded oligonucleotide (SEQ ID NO: 2) moiety, but differ in that the sequence of the double-stranded RNA moiety of the ARNAX120₍₁₇₋₁₃₆₎ is positions 17 to 136 of SEQ ID NO: 1, whereas that of the ARNAX120₍₁₋₁₂₀₎is positions 1 to 120 of SEQ ID NO: 1.

The synthesized nucleic acids were lyophilized and then preserved at -20°C until use. Aggregation formation was analyzed by size exclusion chromatography (SEC-HPLC) under the following conditions.

[SEC-HPLC Analysis Conditions]
Column: TSKgel UP-SW3000, 4.6mm × 300 nm, 2 µm × 2 columns (+ guard column)
Column temperature: 30°C
Mobile phase: 1 x PBS (137 mM NaCl, 2.68 mM KCl, 10 mM Na₂HPO₄, 2 mM KH₂PO₄)
Flow rate: 0.18 mL/min.
Sample diluent: 1 x PBS
Gradient condition: isocratic elution (A 100%)
Detection wavelength: 260 nm

### (1) ARNAX120₍₁₇₋₁₃₆₎

A lyophilized product of ARNAX120₍₁₇₋₁₃₆₎ (before cryopreservation) was dissolved in water for injection at a concentration of 1 mg/mL, and water for injection and 10xPBS were used to prepare the final solution in 1xPBS. The formation of aggregates was analyzed by size exclusion chromatography (SEC-HPLC) (initial). In addition, the formation of aggregates in solutions of ARNAX120(17-136) was analyzed by SEC-HPLC, in samples of a lyophilized product that had been stored at -20°C for one month and then reconstituted, with the measurement performed immediately after reconstitution (1 month freeze), as well as samples subjected to repeated freeze-thaw cycles (freeze-thaw, repeat).

The aggregate percentage of the ARNAX120₍₁₇₋₁₃₆₎ before the cryopreservation was 18%, whereas the aggregate percentage of the ARNAX120₍₁₇₋₁₃₆₎ after the cryopreservation was 35%, and the aggregate percentage of the ARNAX120₍₁₇₋₁₃₆₎ after repeated freeze-thaw cycles was 36.2% (Figure 2). For the ARNAX120₍₁₇₋₁₃₆₎, it was observed that the aggregation was increased by the cryopreservation and repeated freeze-thaw cycles.

### (2) ARNAX120₍₁₋₁₂₀₎

A lyophilized product of ARNAX120₍₁₋₁₂₀₎ was cryopreserved (-20°C), and the formation of aggregates was analyzed by SEC-HPLC (before freezing, and months 1, 3, 6, 9, and 12 after preservation; Figure 3 and Table 1). Further, for the ARNAX120₍₁₋₁₂₀₎ after dissolved in saline and preserved at -20°C, the formation of aggregates was similarly analyzed by SEC-HPLC (months 1 and 3 after preservation) (Table 1). For the cryopreserved lyophilized product of ARNAX120₍₁₋₁₂₀₎, aggregate formation was not observed and the content of the double-stranded active body was constant for 12 months at -20°C. Also for dissolution in saline, stability for one month was confirmed (Figure 3 and Table 1).

**[Table 1]**

| Area percentage | Lyophilized product | Saline |
|---|---|---|
| 0 | 90.3% | 90.1% |
| 1 | 92.0% | 90.0% |
| 3 | 90.7% | 89.0% |
| 6 | 90.2% | |
| 9 | 90.5% | |
| 12 | 90.3% | |

| | | |
|---|---|---|
| Area percentage: percentage of the peak area attributable to the main component (the double-stranded active entity). | | |

### 2. Tm Value

Tm values of ARNAX120₍₁₇₋₁₃₆₎ and ARNAX120₍₁₋₁₂₀₎ were measured. Each nucleic acid was dissolved in 10 mM sodium phosphate buffer (pH 7.4), left to stand for 60 minutes at 5°C, and then heated to 95°C at a rate of +0.5°C/min. 100 µL of each nucleic acid solution (1 mg/mL) was taken and diluted up to 5 mL with the buffer, and then the Tm value was measured.

While the ARNAX120₍₁₇₋₁₃₆₎ showed three stages of structural change, only two stages of structural change were observed in the ARNAX120₍₁₋₁₂₀₎ (Figure 4). It was assumed that the ARNAX120₍₁₋₁₂₀₎ was easy to handle, as it had no structural change around room temperature.

### Example 2: Activity (ability of activating IFNβ via TLR3) of ARNAX120₍₁₇₋₁₃₆₎ and ARNAX120₍₁₋₁₂₀₎ after Freezing and Thawing

### 1. Activity after Freezing and Thawing

HEK293 cells were transfected with a control plasmid (pEF/BOS) or a human TLR3 expression plasmid, together with an IFN-β promoter reporter plasmid, and the ARNAX120₍₁₇₋₁₃₆₎ or ARNAX120₍₁₋₁₂₀₎ was added after 24 hours. The luciferase activity in the cells was measured after six hours. The luciferase activity was expressed as a fold relative to the luciferase activity in the negative control, where a culture medium was added to the pEF/BOS-expressing cells, as 1.

### (1) ARNAX120₍₁₇₋₁₃₆₎

The ARNAX120₍₁₇₋₁₃₆₎ was dissolved in water for injection to a concentration of 1 mg/mL. The sample immediately after the dissolution was added to the cells at a concentration of 10 µg/mL, and the luciferase activity was measured as described above. Similarly, the dissolved sample was frozen and thawed and added to the cells at a concentration of 10 µg/mL, and the luciferase activity was measured. As compared to the sample immediately after the dissolution, the freeze-thawed sample showed only about 65% activity (Figure 5).

### (2) ARNAX120₍₁₋₁₂₀₎

In water: The ARNAX120₍₁₋₁₂₀₎was dissolved in water for injection to a concentration of 1 mg/mL. The sample immediately after the dissolution was added to the cells at concentrations of 1, 5, and 10 µg/mL, and the luciferase activity was measured. Similarly, the dissolved sample was frozen and thawed and added to the cells at concentrations of 1, 5, and 10 µg/mL, and the luciferase activity was measured.

In saline: The ARNAX120₍₁₋₁₂₀₎ was dissolved in saline to a concentration of 1 mg/mL. The solution was then incubated for one hour at 30°C. The dissolved sample was added to the cells at concentrations of 1, 5, and 10 µg/mL, and the luciferase activity was measured. Similarly, the dissolved sample was frozen and thawed and added to the cells at concentrations of 1, 5, and 10 µg/mL, and the luciferase activity was measured.

The ARNAX120₍₁₋₁₂₀₎ showed a concentration-dependent luciferase activity, whether it was dissolved in water or saline (Figure 6). Further, no decrease in the luciferase activity was observed due to the freezing and thawing.

### 2. Activity of Lyophilized Product after Preservation

The lyophilized ARNAX120₍₁₋₁₂₀₎ was preserved for 0 to 12 months at -30°C, and the activity was measured using the same method as in above 1. As a result, the ARNAX120₍₁₋₁₂₀₎ was confirmed to have a stable activity (ability of activating IFNβ via TLR3) for one year when preserved at -30°C (Figure 7).

### Example 3: Influence of Freezing and Thawing on Antitumor Activity of ARNAX120₍₁₋₁₂₀₎

### 1. Tumor Regression Effect of ARNAX120₍₁₋₁₂₀₎

C57BL/6J mice (female, 7 weeks old) were subcutaneously implanted with 2x10⁶ cells of murine tumor strain EG7 (OVA-expressing EL4 lymphoma). After seven days, the animals were confirmed for the tumor volume, and were subcutaneously injected at two sites (right flank and left flank) with 100 µL of ARNAX120₍₁₋₁₂₀₎ (10 µg) + OVA(100 µg)/in 200 µL PBS. The tumor volume was measured every two or three days until day 15. Further, on day 15, tumors were sampled, and the percentages of CD8⁺ T cells (CD3⁺ + CD8⁺ cells) in the tumor and the spleen, and the percentage of OVA-specific CD8⁺ T cells (Tetramer⁺ cells) among the CD8⁺ T cells were measured by flow cytometry.

The tumor volume was 700 mm³ on average on day 7, but was significantly reduced by the administration of the ARNAX120₍₁₋₁₂₀₎ (Figure 8). It was also confirmed that the ARNAX120₍₁₋₁₂₀₎ induced CTLs in the tumor and the spleen, thereby causing tumor regression (Figure 9).

### 2. Influence of Freezing and Thawing on Antitumor Activity

The tumor regression effect of the ARNAX120₍₁₋₁₂₀₎ prepared as in above 1 was compared between a freeze-thawed sample (FT: freeze and thaw, once) and a sample not freeze-thawed (Non-FT: Non-freeze and thaw). Regardless of whether or not they were freeze-thawed, the ARNAX120₍₁₋₁₂₀₎ showed a significant tumor regression effect, and it was confirmed that its antitumor activity was not affected by freezing and thawing (Figure 10).

### Example 4: Antitumor Effect of ARNAX120₍₁₋₁₂₀₎ (Abscopal Effect)

In accordance with Example 3, C67BL/6 mice (female, 7 weeks old) were subcutaneously grafted with EG7 (2x10⁶ cells/200 µL PBS) in the right flank (treatment area), and subcutaneously grafted with EG7 (2x10⁵ cells/200 µL PBS) in the left flank, which is an abscopal area. On day 6, it was confirmed that the tumor volume was 250 to 300 mm³, and the treatment area was irradiated (15 Rad). On the next day, PBS and the ARNAX120₍₁₋₁₂₀₎ (25 µg/200 µL PBS) were subcutaneously administered, and the tumor growth was measured over time in the treatment area and the abscopal area.
No rad/PBS group (n=6)
Rad/PBS group (n=5)
Rad/ARNAX120₍₁₋₁₂₀₎ group (n=5)

The administration of the ARNAX120₍₁₋₁₂₀₎ induced the abscopal effect in which tumors regressed not only in the irradiated area (treatment area) but also in the unirradiated areas (abscopal areas), and extended the survival of the mice as compared to the mice treated by radiotherapy alone (Figure 11).

### Example 5: Cytokine Induction by ARNAX120₍₁₋₁₂₀₎

C57BL/6J mice (7 weeks old, female) were divided into three groups (four mice per group), and were subcutaneously administered with 200 µL saline, 50 µg ARNAX120₍₁₋₁₂₀₎/200 µL saline, or 50 µg poly(I:C)/200 µL saline in a part near the inguinal lymph node. Blood was sampled from the tail vein after three hours and six hours, and serum IL-6, TNF-α, and IL-12p40 were measured by Cytometric Bead Assay (CBA), and serum IP-10 was measured by ELISA (Figure 12).

C57BL/6J mice (8 weeks old, female) were divided into three groups (four mice per group), and were intraperitoneally administered with 200 µL saline, 50 µg ARNAX120₍₁₋₁₂₀₎/200 µL saline, or 50 µg poly(I:C)/200 µL saline. Blood was sampled from the tail vein after three hours and six hours, and serum IL-6, TNF-α, and IL-12p40 were measured by CBA, and serum IP-10 was measured by ELISA (Figure 13).

As compared to poly(I:C), the ARNAX120₍₁₋₁₂₀₎ hardly induced inflammatory cytokines (IL-6 and TNF-α), regardless of the administration route (subcutaneous administration or intraperitoneal administration), but induced Th1 cytokines (IL-12 and IP-10) to the same extent as poly(I:C).

### Industrial Applicability

The nucleic acid of the present invention is easy to handle, has good preservation stability, and maintains a high antitumor activity even after cryopreservation. Therefore, the nucleic acid of the present invention is suitable for practical use as a pharmaceutical product.

All publications, patents, and patent applications cited herein are incorporated herein by reference in their entity.

### Sequence data:

SEQ ID NO:1  diRNA derived from Measles virus ED strain
accagacaaa gcugggaaua gaaacuucgu auuuucaaag uuuucuuuaa uauauugcaa 60
auaaugccua accaccuagg gcaggauuag gguuccggag uucaaccaau uaguccuuaa 120
ucagggcacu guauccgacu aacuuauacc auucuuuggu cuccuugacu guuaccuuaa 180
aaacccacuc acguuucaaa cccccgguca uaauaaucug uuucucugac uuggauagau 240
ucuuaacgaa gauauucugg uguaagucua guaucagaua gccggacuug agauucugga 300
uaaacuuauu uaucaacuuu cuguucccgg aguaaagaag aaugugcccc caaaauuugc 360
gggugauccu agauauaagu ucucguugcc ugcuacuuac caauacgggg uaagcgugga 420
acaucccuug uugacuucuu ugguugucuu ugaaucuugc caacucccug uagaggauga 480
guauagaauu aagcaaucca ucuugcccug aggcaacauc augguggauc aauucuuugc 540
acagcuuagg uccguuaauu gccaacccgc aauugaucag caccugcucu auagguguaa 600
guuuuuucag aguaggauug auaucaccuc uacuaacugc gucucccaca auugcuugua 660
ugcagcuuag uugcuuaaug gauaggaugu gaccuauaag uccaggugaa guccucacag 720
augauucaau uaucugcugc uuaaucuuuu caggauucau uagccgguua gccuugagau 780
cugucauaac caaauaagau ucaguagaua ugaaguugcu guaucuaggg uauacaaggu 840
ucacuucucu auaaugagac ccuacauaac uuauaaaucc cugaacaaaa uccccgcuga 900
aaggcauaag cuuaaucacc aguauugauc cuauuuugcc caggagcaga gccaucgaua 960
agauggcugc caauuccucu agcuucucua uaguaucuuu guuaggcaag uuagucggau 1020
acagugcccu gauuaaggac uaauugguug aacuccggaa cccuaauccu gcccuaggug 1080
guuaggcauu auuugcaaua uauuaaagaa aacuuugaaa auacgaaguu ucuauuccca 1140
gcuuugucug gu 1152

SEQ ID NO:2  single-stranded ODN (ARNAX120(17-136), ARNAX120(1-120))
tgctgctgct tgcaagcagc ttgat 25

SEQ ID NO:3  double-stranded RNA (ARNAX120(1-120))
accagacaaa gcugggaaua gaaacuucgu auuuucaaag uuuucuuuaa uauauugcaa 60
auaaugccua accaccuagg gcaggauuag gguuccggag uucaaccaau uaguccuuaa 120

SEQ ID NO:4  sense strand- chimeric DNA/RNA molecule (ARNAX120(1-120))
tgctgctgct tgcaagcagc ttgataccag acaaagcugg gaauagaaac uucguauuuu 60
caaaguuuuc uuuaauauau ugcaaauaau gccuaaccac cuagggcagg auuaggguuc 120
cggaguucaa ccaauuaguc cuuaa 145

SEQ ID NO:5 antisense strand-synthetic RNA molecule (ARNAX120(1-120))
uuaaggacua auugguugaa cuccggaacc cuaauccugc ccuagguggu uaggcauuau 60
uugcaauaua uuaaagaaaa cuuugaaaau acgaaguuuc uauucccagc uuugucuggu 120

SEQ ID NO:6 double-stranded RNA (ARNAX120(17-136))
aauagaaacu ucguauuuuc aaaguuuucu uuaauauauu gcaaauaaug ccuaaccacc 60
uagggcagga uuaggguucc ggaguucaac caauuagucc uuaaucaggg cacuguaucc 120

SEQ ID NO:7 sense strand- chimeric DNA/RNA molecule (ARNAX120(17-136))
tgctgctgct tgcaagcagc ttgataauag aaacuucgua uuuucaaagu uuucuuuaau 60
auauugcaaa uaaugccuaa ccaccuaggg caggauuagg guuccggagu ucaaccaauu 120
aguccuuaau cagggcacug uaucc 145

SEQ ID NO:8 antisense strand-synthetic RNA molecule (ARNAX120(17-136))
ggauacagug cccugauuaa ggacuaauug guugaacucc ggaacccuaa uccugcccua 60
ggugguuagg cauuauuugc aauauauuaa agaaaacuuu gaaaauacga aguuucuauu 120

## Claims

1. A nucleic acid comprising: a sense strand set forth in SEQ ID NO: 4 and an antisense strand set forth in SEQ ID NO: 5.

2. The nucleic acid according to claim 1, wherein at least a part of nucleotides constituting a single-stranded oligodeoxynucleotide of the sense strand is phosphorothioate-modified.

3. An adjuvant composition comprising the nucleic acid according to claim 1 or 2.

4. A pharmaceutical composition comprising the nucleic acid according to claim 1 or 2.

5. The pharmaceutical composition according to claim 4, wherein the composition is used in combination with radiotherapy.

6. The pharmaceutical composition according to claim 4, wherein the composition is used in combination with an immune checkpoint inhibitor.

7. The pharmaceutical composition according to claim 4, further comprising an antigen.

8. The pharmaceutical composition according to any one of claims 4 to 7, for treating or preventing cancer or an infectious disease.
